# EUROPEAN PATENT APPLICATION

(11) **EP 4 342 996 A1**
(43) Date of publication of application: **27.03.2024**
(21) Application number: 22197010.6
(22) Date of filing: 22.09.2022
(51) Int. Cl.: C12Q 1/00, C12Q 1/26

(54) **ALFA-AMYLASE ACTIVITY BIOSENSOR**

(71) Applicant: Bioanalizes sistemos, UAB, 10224 Vilnius (LT)
(72) Inventor: Butkevicius, Marius, Vilnius (LT); Gureviciene, VIdute, Vilnius (LT); Razumiene, Julija, Vilnius (LT); Laurynenas, Audrius, Vilnius (LT); Dagys, Marius, Vilnius (LT)
(74) Representative: Pakeniene, Ausra

(57) **Abstract**

This invention is an enzyme-based biosensor for α-amylase activity determination. The α-amylase activity determination is based on reagentless oxidation of maltose, which is formed during an α-amylase-catalyzed reaction. Maltose oxidation is catalyzed by PQQ-GDH which is immobilized on a membrane disposed on a removable O-ring. This biosensor is suitable for α-amylase activity determination in various water-based solutions.

## Description

### TECHNICAL FIELD

The invention relates to an enzyme-based biosensor for assaying α-amylase directly by maltose formed in enzyme-catalyzed hydrolysis of starch or other suitable substrate.

### BACKGROUND ART

α-amylases are endo-acting enzymes that catalyze the initial hydrolysis of starch and related carbohydrates into shorter oligosaccharides through the cleavage of internal α-D-1,4-glycosidic bonds with the retention of α-anomeric configuration in the products. In starch hydrolysis, α-amylase act at random locations of the substrate molecule, yielding oligosaccharides with different chain length, which constitute a mixture of maltose, maltotriose, and branched oligosaccharides of six to eight glucose molecules that contain both α-1,4 and α-1,6 linkages. Theoretically, there is no production of glucose during this process.

In humans, α-amylase is one of the major secretory products of the pancreas and salivary glands, playing a role in digestion of starch and glycogen. The determination of α-amylase activity in serum, urine or other body fluids is widely used in the clinical laboratory for diagnosis of pancreatic or salivary glands disorders, e.g., acute pancreatitis or parotitis.

There are several methods reported in the literature to measure α-amylase activity in varies solution (e.g., enzymatic method, reducing sugar method, chromogenic method). For these methods, different substrates are used including starch, amylose, amylopectin, and some chemically modified derivatives of polymers and malto-oligosaccharides of varying chain length linked to a chromophore, such as 4-nitrophenyl or 2-chloro-4-nitrophenyl. Though several techniques are reported, the most common technique employed in the clinical and commercial field is spectrophotometry.

The most common reagent for reducing methods is 3,5-dinitrosalicylic acid (DNSA). All reducing methods are based on measuring the oxidizing ability of reducing sugars. Sugar molecules act as reducing agents as long as they contain an aldehyde group and exist in an open-chain structure. Monomeric sugars exist in aqueous solution in equilibrium between their open-chain and ring structures, but only the open structures are responsible for their reducing activity. During dimer formation, the aldehyde group of one of the sugars is buried in the glycosyl bond, making it incapable of acting as a reducing agent. There are different techniques of reducing sugar methods, but it always consists of a few steps (recently 6-8 steps) and often involves heating and cooling.

Chromogenic methods are based on interaction of the substrate with α-amylase and release of the chromophore upon hydrolysis by a α-glucosidase. Most often a soluble dye or a chromogen is coupled to a substrate (e.g., malto-oligosaccharides) to give a measurable optical signal when the chromogen is liberated as a result of α-amylase action.

European patent application EP0309256 A2 discloses a variant of a chromogenic method where this method is applied to electrochemical measurements (see). In electrochemical measurements, the chromogenic group is replaced by a redox active compound (e.g., p-aminophenol).

Enzymatic methods in most cases are based on the interaction of glucose/products of α-amylase with glucose oxidase (GOx) and are used to determine blood glucose level, total starch content in cereal products, and serum α-amylase activity. These methods are cheap, stable, and highly specific for the substrate, D-glucose. Because GOx is highly specific, the product of α-amylase (e. g. maltose and other maltodextrins) cannot be used for direct determination of α-amylase activity. For this reason, an additional enzyme - amyloglucosidase is used to yield glucose. GOx is used along with peroxidase (POD) to determine the amount of glucose produced. The principle of the method is that glucose is oxidized by GOx to yield gluconic acid and hydrogen peroxide. Afterwards, the H₂O₂ generated reacts with an electron acceptor, such as quinoneimine, in the presence of POD to form a pink-coloured product, whoseabsorbance is measured at 500 nm. Enzymatic methods based on GOx are adapted for electrochemical measurements, wherein the produced amount of D-glucose is registered with a glucometer.

Publication Razumiene, J., Vilkanauskyte, A., Gureviciene, V., Barkauskas, J., Meskys, R., & Laurinavicius, V. (2006). Direct electron transfer between PQQ dependent glucose dehydrogenases and carbon electrodes: An approach for electrochemical biosensors. Electrochimica Acta, 51(24), 5150-5156 discloses a biosensor based on PQQ-GDH that can oxidize saccharides containing reducing end including D-glucose, but it is not adapted for detection of α-amylases activity. Furthermoreit has a complex manufacturing methodology.

All the above-mentioned methods have drawbacks. Typically, performing of these methods require several steps, which indicates that trained personnel is needed. Also, some of the methods require additional enzymes (e. g., amyloglucosidase) to produce D-glucose. Moreover, some of the mentioned methods require additional compounds which correlate with more complex waste cleaning. Thus, the above-mentioned facts demonstrate that there are challenges related to the α-amylase activity determination and a simpler detection method is still needed.

The present invention is dedicated to overcoming of the above shortcomings and for producing further advantages over prior art.

### BRIEF DESCRIPTION OF THE INVENTION

The present invention is an α-amylase activity biosensor for determination of α-amylase activity. Determination of amylase activity is based on reagentless enzymatic maltose oxidation by an enzyme for oxidizing maltose, such as PQQ dependent glucose dehydrogenase. An electrochemical signal is recorded during measurements.

The measurement of α-amylase activity is based on electrochemical signals. Direct formation of maltose is registered and does not require an additional enzyme (e.g., glucoamylase). The detection system is localized on an easily replaceable terylene membrane or a permeable membrane.

The method is reusable, does not require trained personnel, the procedure is simple to perform, and no additional equipment or preparation of the sample is required.

### BRIEF DESCRIPTION OF THE DRAWINGS

Features of the invention believed to be novel and inventive are set forth with particularity in the appended claims. The invention itself, however, may be best understood by reference to the following detailed description of the invention, which describes exemplary embodiments, given in non-restrictive examples, of the invention, taken in conjunction with the accompanying drawings, in which:
Fig. 1 shows a schematic measurement curve of samples as measured with the biosensor according to the invention: A - sample with reducing sugar (e.g., D-glucose, maltose), B - sample without reducing sugar. At point 1 the analyte is placed, at point 2 the α-amylase substrate is placed (e.g., starch).
Fig. 2 shows a calibration curve of the biosensor according to the invention. Measurements were performed at 25 °C using 50 mM Tris buffer solution with 100 mM KCl and 5 mM CaCl₂. The working electrode is polarized at 600 mV vs. NHE (Normal hydrogen electrode).

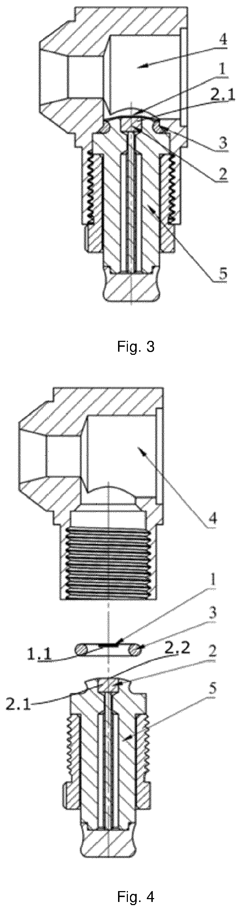

Fig. 3 shows scheme of the fully assembled biosensor according to the invention: a membrane (1) with immobilized enzyme, a graphite working electrode (2), an O-ring (3), a measuring cell (4), a body of working electrode (5).
Fig. 4 shows schematic assembly of the biosensor: a membrane (1) with immobilized enzyme, a graphite working electrode (2), an O-ring (3), a measuring cell (4), a body of working electrode (5).
Preferred embodiments of the invention will be described herein below with reference to the drawings. Each figure contains the same numbering for the same or equivalent element.

### DETAILED DESCRIPTION OF THE INVENTION

It should be understood that numerous specific details are presented in order to provide a complete and comprehensible description of the invention embodiment. However, the person skilled in art will understand that the embodiment examples do not limit the application of the invention which can be implemented without these specific instructions. Well-known methods, procedures and components have not been described in detail for the embodiment to avoid misleading. Furthermore, this description should not be considered to be constraining the invention to given embodiment examples but only as one of possible implementations of the invention.

The presented invention is an α-amylase activity biosensor (BS) for registering direct formation of maltose. The α-amylase activity biosensor comprises a membrane (1) having immobilized enzyme for oxidizing maltose, such as a soluble pyrroloquinoline quinone glucose dehydrogenase (PQQ-GDH) and a working electrode (2), an O-ring (3), a measuring cell (4), a body of working electrode (5).

The membrane (1) is fixed on the O-ring (3), preferably glued and preferably on a rubber O-ring, where the O-ring is disposed at one end on the body (5) of the working electrode (2) on and around the tip (2.1) of the body (5) of the working electrode (2) so that the membrane (1) at its inner surface (1.1) would touch upper surface (2.2) of the working electrode. The body (5) of the working electrode (2) accommodates the working electrode (2) exposing the tip (2.1) of the working electrode (2) to the sample measurement space of the cell (4) where at least part of the working electrode (2) extends through inside of the body (5).

The membrane (1) is preferably a terylene membrane. The working electrode (2) is preferably made of graphene.

When PQQ-GDH is used, PQQ-GDH is immobilized on the membrane (1) using a mixture of bovine serum albumin (BSA), glutaraldehyde, reduce graphene. PQQ-GDH is immobilized by mixing 4 µl reduce graphene solution (10 mg/ml), 2 µl 1 % BSA solution, 2 µl 2.5 % glutaraldehyde solution and 2 µl PQQ-GDH solution (500 U/ml). The entire immobilization mixture is placed on the membrane (1) and left at room temperature, about +25 ºC to dry. After that, the membrane (1) is stored at +4 ºC temperature until it is used.

The membrane (1) facilitates storage, replacement, and transportation of the biosensor (BS). The membrane (1) allows for reducing the size and price of the biosensor (BS) because the enzyme is immobilized on the membrane (1), whose size and shape is easily modified. Further the above advantages are enhanced by the membrane (1) being attached to the O-ring (3) which is detachably disposed on the tip (2.1) of the body (5) for securing the working electrode (2) to the measuring cell (4).

The working electrode (2) is removably disposed in the body (5) of the working electrode (2) and can be used several times by replacing the O-ring (3) with the membrane (1) and therefore reduces the price.

The membrane-based biosensor (BS) according to the invention can be manufactured in one step, due to the following:
i) good adhesion of the enzyme immobilization mixture to the membrane, which occurs due to the hydrophilic properties of the membrane;
ii) homogeneity of the enzyme immobilization mixture, since all components are highly soluble in aqueous solution;
iii) good contact between the working electrode (2) and the membrane (1), which is created using the O-ring (3) by tight fixing the O-ring (3) on to the tip (2.1) and around of the body (5) of the working electrode (2).

Before applying the enzyme-based membrane (1) to the working electrode (2), the working electrode is polished, for example, using sandpaper and thoroughly rinsed with deionized water.

In another embodiment of the invention, variants of graphene, e.g., graphene modification with organic dyes, or carboxylate graphene, or graphene oxide, are used as electrode material.

In yet another embodiment of the invention the working electrode (2) is made of other carbon-based material (e.g., glassy carbon, etc.) or metal (e.g., gold, platinum, brass etc.).

In yet another embodiment of the invention, nanomaterials such as metal-based nanomaterials (nanoparticles, quantum dos, etc.), or carbon-based nanostructures (carbon nanotubes, etc.), or organic polymers (polyaniline, etc..) are used as electrode material.

In yet another embodiment of the invention PQQ-GDH is immobilized using for example adsorption, covalent bonding or other immobilization methods.

The terylene membrane is a permeable membrane such as dialysis membrane, or similar.

α-amylase activity detection is based on reagent-free maltose and/or short maltodextrin, which are formed during the α-amylase-catalyzed reaction, oxidation, as a substrate using starch or a similar compound. The enzymatic activity measurement is carried out in this invention by measuring the current change over time when starch or other α-amylase substrate is added into analyzed solution. The change of current occurs due to a bioelectrochemical reaction catalyzed by PQQ-GDH.

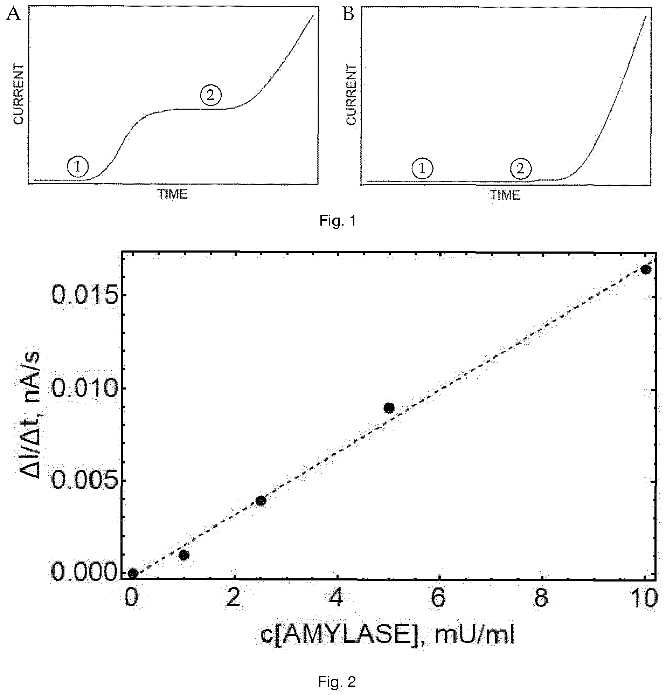

The determination of α-amylase activity comprises:
Measurements are performed after electrochemically polarizing the working electrode to at least 500 mV vs. NHE, but less than 1000 mV vs. NHE. Adding a sample for measuring into a measuring cell (4). After sample addition (Fig. 1), if the sample has a sugar with a reducing end, an increase in current is observed, which is proportional to the sugar concentration. Calibration with a solution of known concentration is required to estimate the approximate concentration. If there is no additional saccharide in the sample there is no increase in current. In both cases, a current is allowed to settle, for 30-90s, after the sample has been added.

Adding α-amylase substrate into a measuring cell (4). For example, soluble starch can be used as an α-amylase substrate. Other substrates such as maltodextrines (n > 14), amylose, can be used.

Addition of starch and the presence of amylase in the solution initiate a hydrolysis reaction to form maltose. Maltose acts as a substrate for PQQ-GDH and an increase in current is observed during the reagentless bioelectrochemical reaction. The rate (current change in time) of maltose oxidation is directly proportional to the concentration of α-amylase. Calibration with a solution of α-amylase of known concentration is carried out before testing. Calibration and measurement time is 600s.

**Examples of use of the** α-amylase activity biosensor according to the invention:

### Example 1

The α-amylase activity biosensor according to the invention was applied for the detection of α-amylase activity in saliva. First, the sensor was calibrated with solutions of the α-amylase of known concentration, the calibration curve is shown in Figure 2. The calibration curves show that the lowest α-amylase concentration that can be determined is 0.001 U/ml. 20 samples of human saliva sample were tested and one α-amylase concentration (0.01 U/ml) was used for sensor calibration. Samples were taken regardless of gender, age, health status, time of day and time after meals. Also, the results were compared with a commercial amylase activity assay kit (Sigma-Aldrich, SKU MAK009-1KT). A good correlation was found between the described method and the commercial method, correlation coefficient was 0.975 (n = 20). Measurements were performed at 25 °C using 50 mM Tris buffer solution with 100 mM KCl and 5 mM CaCl₂. A 50 µl saliva sample and 0.95 mL buffer solution were used. After the sample has been added and steady current achieved, 50 µl 1% starch solution was added

### Example 2

The α-amylase activity biosensor according to the invention was applied for the detection of α-amylase activity in human urine or/and blood serum to detect acute pancreatitis. Increase of α-amylase in human urine and blood serum have been observed in acute pancreatitis. Urine sample was assayed in the same manner as in Example 1, a good correlation was obtained between the proposed method and the commercial method.

Although numerous characteristics and advantages together with structural details and features have been listed in the present description of the invention, the description is provided as an example fulfilment of the invention. Without departing from the principles of the invention, there may be changes in the details, especially in the form, size and layout, in accordance with most widely understood meanings of the concepts and definitions used in claims.

## Claims

1. An α-amylase activity biosensor comprising a working electrode and an immobilized enzime **characterised in that** the α-amylase activity biosensor further comprises a membrane (1) covering at least part of the working electrode (2) and in contact with the working electrode (2),
wherein the membrane (1) comprises the immobilized enzyme for oxidizing maltose,
the biosensor further comprises a removable O-ring (3), a measuring cell (4) and a body (5) of the working electrode (2),
wherein the membrane (1) is disposed on the removable O-ring (3),
wherein the removable O-ring (3) with the membrane (1) is disposed on a tip (2.1) and around the tip (2.1) of the body (5) of the working electrode (2) so that the membrane (1) is situated right above the working electrode (2) and in contact with the working electrode (2) and in the measuring cell (4).

2. The α-amylase activity biosensor according to claim 1, wherein the immobilized enzyme is any oxidoreductase, which oxidize maltose or other short reducing sugars.

3. The α-amylase activity biosensor according to claim 1 or 2, wherein the immobilized enzyme is PQQ-GDH.

4. The α-amylase activity biosensor according to any one of claims 1 - 3, wherein the membrane (1) is a permeable membrane.

5. The α-amylase activity biosensor according to any one of claims 1 - 4, wherein the membrane (1) is a terylene membrane.

6. The α-amylase activity biosensor according to any one of previous claims 1-5, wherein the membrane (1) further comprises nanomaterial such as reduced graphene or metal-based nanoparticles.

7. The α-amylase activity biosensor according to any one of previous claims 1-6, wherein the membrane (1) comprises a crosslinking agent for enzyme immobilization, selected from aldehydes, carbodimides, imidoesters, pyrocarbonates.

8. The α-amylase activity biosensor according to any one of previous claims 1-7, wherein the working electrode (2) material is selected from carbon, graphene, brass, silver, platinum, and gold.
